# EUROPEAN PATENT APPLICATION

(11) **EP 1 953 134 A1**
(43) Date of publication of application: **06.08.2008**
(21) Application number: 07001946.8
(22) Date of filing: 30.01.2007
(51) Int. Cl.: C07C 43/15, C07C 45/45, C07C 45/51

(54) **Process for preparing dienones**

(71) Applicant: DSM IP Assets B.V., 6411 TE Heerlen (NL)
(72) Inventor: Bonrath, Werner, 79115 Freiburg (DE)
(74) Representative: Teipel, Stephan

(57) **Abstract**

The present invention relates to a process for preparing dienone, in particular 6-methyl-octa-4,5-dien-2-on and 6-methyl-octa-3,5-dien-2-on, and intermediates in this process.

## Description

The present invention relates to a process for preparing dienones and intermediates useful in preparing dienones.

Dienones, in particular 6-methyl-octa-3,5-dien-2-on and 6-methyl-octa-4,5-dien-2-on are interesting compounds for the flavour and fragrances industry.

Processes for the synthesis of dienones are known, which are based on pyrolysis of tertiary-acetylenic carbinyl acetoacetates.

US 741,047 discloses a process for the production of a doubly unsaturated ketone which comprises pyrolysing a tertiary acetylenic carbinyl acetonate in the presence of an acidic catalyst. The acetoacetates are prepared by the reaction of the corresponding carbinols with diketen.

DE 1 078 112 discloses a process for the preparation of dienones, wherein esters from the general formula of tertiary acetylenic carbinyl acetoacetates are pyrolized, wherein the pyrolysis is conducted in the presence of lower aluminiumtrialcoholates and a lower fatty acid.

However, the known processes for the preparation of dienones have the disadvantages of low yields, complicated chemistry such as handling of the aluminium compounds, and the waste formation, such as decarboxylation during pyrolysis.

Therefore, there is still a need for further methods of synthesizing dienones, in particular 6-methyl-octa-3,5-dien-2-on (1) and 6-methyl-octa-4,5-dien-2-on (2), which avoid these prior art problems. In particular there is a need for a process for the preparation of dienones, which provides dienones in high yield and high purity.

It has now been found that dienones (1) and (2) can be advantageously synthesized according to the following reaction scheme 1:

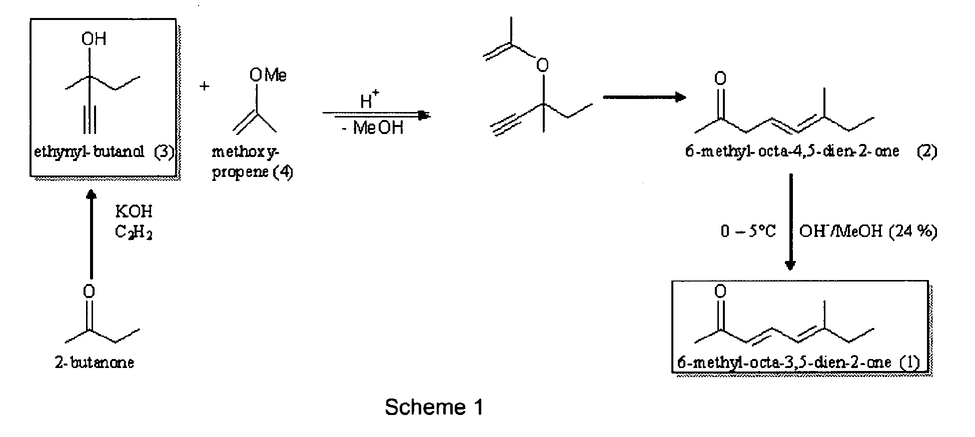

The advantage of this scheme is that the dienones (1) and (2) can be synthesized in excellent yield and purity, in particular as the high temperatures as needed for pyrolysis have not to be applied.

Therefore, the present invention relates to a process for the preparation of a compound of the formula II 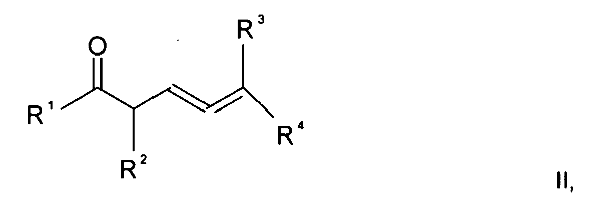 wherein R¹ is a C₁- C₂₅ hydrocarbon moiety, and R², R³ and R⁴ are independently from each other H or a C₁ - C₂₅ hydrocarbon moiety, which process comprises the step of reacting a compound of the formula III 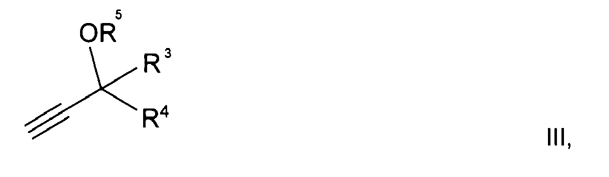 wherein R³ and R⁴ are defined as above and R⁵ is H,
with a compound of the formula IV 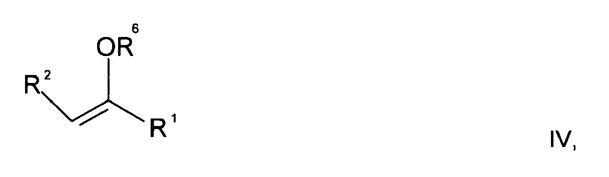 wherein R¹ and R² are defined as above and R⁶ is a hydroxy protecting group.

As C₁ - C₂₅ hydrocarbon moiety of residues R¹, R², R³ and R⁴ each branched or non branched alkyl, branched or nonbranched cyclo-alkyl, branched or nonbranched alkenyl, branched or nonbranched cyclo-alkenyl, or aryl group, which can optionally be substituted, can be present. Preferably, the hydrocarbon moiety is a straight, branched or cyclic C₁ - C₁₆ alkyl or a straight, branched or cyclic C₂ - C₁₆ alkenyl or a C₆ - C₁₆ aryl, such as phenyl or naphtyl, which's aryl may optionally be substituted by an alkyl group. More preferably, R¹ is a C₁ - C₆ alkyl or a C₂ - C₆ alkenyl, in particular methyl, ethyl, n-propyl, isopropyl, n-butyl, isobutyl, tertbutyl, n-pentyl and n-hexyl and R², R³ and R⁴ are a hydrogen or a C₁ - C₆ alkyl or a C₂ - C₆ alkenyl, in particular methyl, ethyl, n-propyl, isopropyl, n-butyl, isobutyl, tertbutyl, n-pentyl and n-hexyl. Most preferred, R¹ is methyl or ethyl, in particular methyl, R² is hydrogen, methyl or ethyl, in particular hydrogen, R³ is hydrogen or methyl, in particular methyl, and R⁴ is methyl, ethyl or n-propyl, in particular ethyl.

In one preferred embodiment of the processes of the present invention, residues R³ and R⁴ are different hydrocarbon moieties and the doublebond including the carbon atom R³ and R⁴ are attached to is preferably in E-configuration. Most preferably, R³ is methyl and R⁴ is ethyl.

R⁵ is hydrogen and R⁶ is a hydroxy protecting group. As protecting groups for the protected hydroxy group usual protecting groups known to the person skilled in the art may be used. Suitable protecting groups are exemplified in WO 03/044011, the content of which is incorporated by references herein. A preferred protecting group for R⁶ is a methyl group.

The reaction of the compound of the formula III with the compound of the formula IV of the process for the preparation of a compound of the formula II of the present invention can be carried out in any usual solvent, which preferably provides sufficient solubility to the reducts used, e.g. C₁ to C₁₂ hydrocarbons. However, the reaction of the compound of the formula III with the compound of the formula IV of the process for the preparation of a compound of the formula II of the present invention is preferably carried out without solvents. The reaction can be carried out under acid catalysis. As suitable acid any preferably strong acid can be used, preferably a strong inorganic acid, e.g. phosphoric acid or sulforic acid, the latter being particularly preferred. The reaction is preferably carried out in a temperature range of 90°C to 150°C, more preferably 100°C to 130°C, in particular at about 105°C, depending on the specific educts used. The reaction can be carried out under inert atmosphere (e.g. argon atmosphere) and is preferably carried out under increased atmospheric pressure, e.g. in a pressure range of 2- 10 bar absolute, in particular 3 to 7 bar absolute.

The compound of formula II, which is preferably obtained by the process for the preparation of compound of formula II of the present invention, can advantageously be used to obtain a compound of the formula I by isomerisation.

Therefore, the process of the present invention preferably further comprises the step of isomerising a compound of formula II 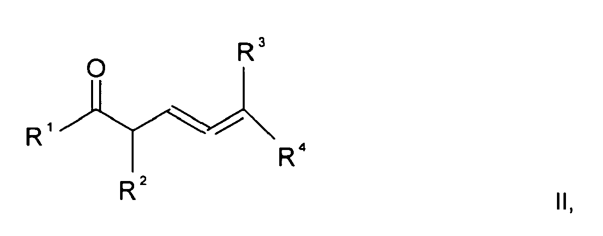 wherein R¹, R², R³ and R⁴ are defined as above to obtain a compound of formula I 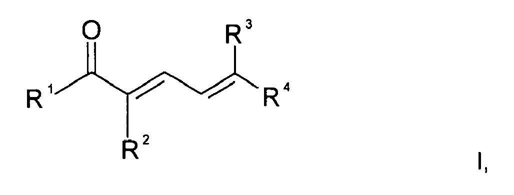 wherein R¹, R², R³ and R⁴ are defined as above.

The isomerisation reaction for the preparation of a compound of formula I can be carried out in any suitable solvent, e.g. water, alcohol such as methanol or ethanol or esters such as ethylacetat. The preferred solvent is methanol. Preferably the isomerisation reaction is catalyzed by an alkaline compound. As suitable alkaline compound hydroxide containing compounds such as NaOH, KOH or similar can be used, preferably NaOH is used. The isomerisation reaction is preferably carried out in a temperature range of -20°C to +20°C, e.g. at about 0°C. The isomerisation reaction can be carried out under inert atmosphere, such as argon atmosphere. The product, i.e. the compound of the formula I, can be purified according to methods known in the art, preferably the compound of the formula I is extracted from the raw reaction product with an organic solvent, e.g. methyltertbutylether (MTBE). The product can be further purified, e.g. by distillation.

In one embodiment of the process of the present invention the compound of formula II is obtained as described above and isomerised to obtain a compound of formula I. The compound of formula II can be purified before the isomerisation reaction to obtain the compound of formula I is conducted. However, more preferred the compound of formula II is isomerised to obtain a compound of formula I without prior purification, i.e. the raw product from the process of the present invention to prepare the compound of formula II is subjected to an isomerisation reaction as described above to obtain the corresponding compound of formula I.

In the most preferred embodiment of the process of the present invention R¹ is methyl, R² is H, R³ is methyl and R⁴ is ethyl, such that the compound of the formula II is 6-methyl-octa-4,5-dien-2-on (2) and the compound of the formula I is 6-methyl-octa-3,5-dien-2-on (1).

According to the processes of the present invention dienones, in particular 6-methyl-octa-4,5-dien-2-on (2) and 6-methyl-octa-3,5-dien-2-on (1) can be advantageously obtained in excellent yields and in excellent purity.

The present invention further relates to the compound of formula V 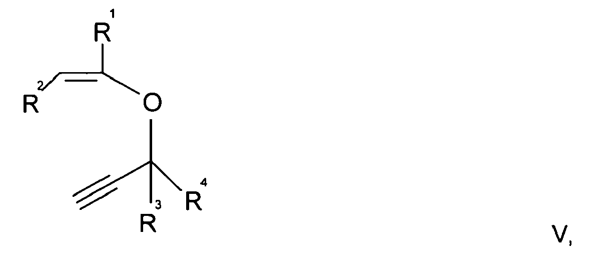 wherein R¹, R², R³ and R⁴ are defined as above.

The compounds of formula V are useful in the preparation of dienones.

The compound of formula V wherein R¹ is methyl, R² is hydrogen, R³ is methyl and R⁴ is ethyl is useful in the preparation of 6-methyl-octa-4,5-dien-2-on (2) and in the preparation of 6-methyl-octa-3,5-dien-2-on (1).

Within this application, all educts, intermediates and products to be used in the process of the present invention can be used as racemates or enantiomerically enriched mixtures, e.g. mixtures which are enriched in one enantiomer or comprise only one substantially purified enantiomer.

Each process of the present invention can further comprise one or more steps of separation or enrichment of enantiomers, e.g. steps of racemic separation. Methods of separation or enrichment of enantiomers are known in the art.

Preferably, the stereoconfiguration of reducts, intermediates and products is chosen such that when used in processes for the present invention, the intermediates and products resulting from said processes show the stereoconfiguration suitable for the preparation of 6-methyl-octa-4,5-dien-2-on and 6-methyl-octa-3,5-dien-2-on.

The present invention will now be further illustrated by the following examples which are not intended to be limiting.

### Example 1: 6-methyl-octa-4,5-dien-2-on (2) (compound of formula II)

### Ketonization, with sulphuric acid as catalyst on a small scale in a 33 ml autoclave

3.63 g EBI (99.5 area%), 36.8 mmol, and 51.7 mg H₂SO₄ (6.97 w% in methanol), = 0.1 mol%, are weighed in to a 33 ml Hastelloy C4 autoclave. 8.24 g IPM (96.5 area%), 110.3 mmol = 3 eq, are weighed to this mixture and then the reactor was immediately closed and placed in the aluminum heating block of the lab-shaker LSR/L-V (Adolf Kühner AG, Birsfelden). The same procedure was done for three other autoclaves.
The shaker frequency was set to 250 min⁻¹, the reaction temperature was 105 °C, the reaction time was 120 minutes (incl. 15 minutes heating-up)
After reaction all the autoclaves were cooled down to room temperature in an ice-bath, opened and neutralized with sodium-acetate. The 4 reaction mixtures were united, solved in 200 ml MTBE and 3 times extracted with 30 ml water deionized (total 120 ml) to wash out the DMP. All water phases were rewashed with 30 ml MTBE. The united organic phases were dried with Na₂SO₄, filtered and concentrated under vacuum at 40°C and 10 mbar. The crude product was distilled bulb-to-bulb under vacuum. The product fraction was taken at T_{J} (Temperature Jacket) 105 °C and 8 mbar. Yield was 19.5 g of a yellowish oil, analyzed by GC area%, GC MS, IR, MA and NMR (NMR-data is shown below).
GC area: 93% (area%)
GC MS M⁺ = 138
IR peaks: 2967.44, 2934.18, 2904.98, 2877.57, 2853.32, 1964.94, 1714.51, 1622.79, 1572.04, 1454.78, 1421.74, 1397.39, 1355.56, 1329.85, 1308.55, 1269.86, 1231.31, 1218.02, 1155.48, 1065.23, 1051.75, 1004.47, 966.99, 924.6, 870.42, 821.96, 787.47, 752.95.
MA
Calcul. C: 78.21 H: 10.21
Found C: 77.41/77.08 H: 10.16/9.87

### Example 2: 6-methyl-octa-4,5-dien-2-on (2) (compound of formula II)

### Ketonization, at ambient pressure on a larger scale in a 5 liter double-walled reactor

1100 g EBI (99.5 area%), 11.152 mol, and 15.69 g H₂SO₄ (6.97 w% in methanol), = 0.1 mol%, were mixed in a 5I double-walled reactor connected to a julabo thermostat F32 (filled with syltherm oil XLT) and equipped with a PT 100, 2 stainless steel intermig stirrers (Ekato) and two condensers each 30 cm long. The reactor system was overlaid with argon. At room temperature and stirring (250rpm) 2500 g IPM (96.5 area%), 33.457 mol = 3 eq were added to this mixture. The internal temperature was rising during this process up to 40 °C. The jacket temperature (T_{J}) was first set to 60 °C and then within 30 minutes slowly increased (intense reflux) up to 70 °C. Within 8 hours the internal temperature increased from 50 °C up to 70 °C. During the 12 hour after reaction time the color of the reaction mixture turned from light yellow to amber. The reaction mixture was actively cooled down to room temperature. For the work-up 2 liters of MTBE were added to the reaction mixture and then extracted 3 times with 500 ml water deionized (total 1.5 liters). The MTBE is necessary to separate the phases, because the water is soluble in DMP. The water phase was not re-extracted with MTBE (results in a one phase system). The organic phase was dried with Na₂SO₄, filtered and concentrated under vacuum at 40°C and 10 mbar. The crude product was distilled in a 2 liter two-necked round-bottomed flask with an oil bath, magnetic stirrer, PT 100, 30 cm Vigreux-column, Liebig-condenser, two-way fraction-separator, cold trap, membrane vacuum-pump. The first product fraction was taken at T_{J} 74 - 75°C, internal temperature (T_{I}) 64 - 66°C, distillate temperature (head) (T_{H}) 59 - 60°C and 8 mbar.

### Example 3: 6-methyl-octa-4,5-dien-2-on (2) (compound of formula II)

### Ketonization on a larger scale in a 1 liter autoclave

| | a) | b) | c) | d) |
|---|---|---|---|---|
| internal temperature [°C] T_{I} | 105 | 150 | 105 | 95 |
| eq IPM | 3 | 3 | 2,5 | 2,5 |
| Mol-% H₂SO₄ (6,97 w% in MeOH) | 0,1 | 0,1 | 0,1 | 0,1 |

In experiments a) - d) a stainless steel batch reactor from Medimex - High Pressure, number 910470 MED. 243 (build 1995), with a nominal volume of 1.0 liter, a operative temperature up to 220 °C and a maximum pressure of 20 bar was used. The heating system of the reactor is provided by electrical heating spirals located in the jacket. Cooling is obtained by water flowing through the reactor jacket. The temperature control comprises three thermocouples for the measurement of the inner reactor, jacket and cooling temperatures, with precision of ± 0.05 °C. A sensor measures the pressure with a precision of ± 0.05 bar. The stirrer is a stainless steel four-propeller with a speed range from 0 to 1200 rpm, from Flender ATB-Loher, number EAFY63/2B-7/N12 (0.25 kW). The sampling was done via a stainless steel capillary and a thin spiralled stainless steel tube connected to the sampling flask. The reactor is also connected to a safety and a data control system.

1.7 g of the catalyst H₂SO₄ (6.97 w% in methanol), =0.1 mol% were solved in 120.9 g EBI (99.5 area%), 1.23 mol and poured in to the reactor. 274.7 g IPM (96.5 area%), 3.68 mol = 3 eq were added. Total volume in all experiments was 500 ml. The reactor was immediately closed and the stirrer was started (Set on 500 rpm). The reaction mixture was heated up to set temperature (T_{I}) and then, the experiment was carried out at isothermal conditions. The sampling was done by using the stainless steel capillary connected to an ice cooled, thin-spiralled, stainless steel tube. The collected sample was directly neutralized with sodium acetate. The sample was analyzed by gas chromatography (area%). After the reaction time, the reaction mixture was cooled to 25 °C. A sample of the reaction mixture was collected through the sample valve and capillary by introducing low-pressure nitrogen in to the reactor. The conversion time, conversion rate and the obtained yields of examples a) to d) are summarized in the following table.

| | | **a)** | **b)** | **c)** | **d)** |
|---|---|---|---|---|---|
| Time | [Min] | 115 | 60 | 150 | 210 |
| Conversion | 1%] | 100 | 100 | >95 | >95 |
| Yield | [%] | >95 | >95 | >95 | >95 |

| | | | | | |
|---|---|---|---|---|---|
| Conversion and yield based on area%. | | | | | |

### Example 4: 6-methyl-octa-3,5-dien-2-on (1) (compound of formula I)

### Isomerisation of (2) with NaOH (aq.) in methanol on a small scale

A 25 ml two-necked round-bottomed flask equipped with magnetic stirrer, PT 100, dropping funnel, condenser with argon overlay and dry-ice/alcohol bath was used. Between 0 and 4 °C and over 45 minutes 8 g 6-methyl-octa-3,4-dien-2-on (2) (92.97 area% = 53 mmol) were drop wise added to a mixture of 86.5 mg NaOH (aq.) 24w% in water deionized = 0.52 mmol and 2.9 g methanol. The reaction was very exothermic and had to be controlled with the dry-ice/alcohol bath. There is a change in color from light yellow to amber. The solution was neutralized with 29 mg acetic acid (= 0.48 mmol) which caused a discoloration. For the work-up the low-boilers were evaporated at T_{J}40 ° and down to 10 mbar. The residue was solved in 50 ml MTBE and extracted once with 2.9 g water deionized. After separation the water phase was re-extracted with 10 ml MTBE. The united organic phases were dried with Na₂SO₄, filtered and concentrated under vacuum at 40°C and down to 10 mbar. The crude product was distilled bulb-to-bulb under vacuum. The product fraction was taken at T_{J} up to 105 °C and 9 mbar.
Yield :7.13 g of a yellowish oil, analyzed by GC area%, GC MS, IR, MA and NMR. (NMR-data is shown below).
GC 95.4% E/Z + E/E 6-methyl-octa-3,5-dien-2-on (1) (obtained E/Z to E/E ratio - 58:42)
GC MS: M⁺ = 138
IR peaks: 3042.89, 2968.65, 2936.45, 2877.12, 1683.97, 1667.13, 1627.47, 1586.08, 1445.58, 1428.33, 1377.39, 1359.20, 1309.04, 1272.26, 1252.62, 1207.9, 1165.83, 1142.38, 1062.58, 1015.36, 1000.53, 971.08, 884.87, 829.91, 790.42, 768.32, 622.47.
MA:
Calcul. C: 78.21 H: 10.21
Found C: 76.99/76.90 H: 10.11/10.16

### Analytics of examples 1 and 4

### 1. NMR data

NMR spectra were recorded in CDCl₃ on a Bruker Avance 300 spectrometer equipped with 5 mm BBO BB-1 H probe head. NMR data are given relative to TMS: δ_{C} 0.0 ppm, δ_{H} 0.00 ppm, (Tables 1 and 2). Assignments were based on extensive 1 D and 2D NMR experiments, including NOESY, HSQC and HMBC.

### Compound 1: 6-Methyl-octa-4,5-dien-2-one (example 1)

Characteristic ⁵*J* coupling constants in the order of 3 Hz between H-4 and both CH₂-7 and CH₃-6 indicating an allene moiety were observed.

### Compound 2: (3E,5E)-6-Methyl-octa-3,5-dien-2-one (example 4)

The large value of *J*_{H3-H4} (15.3 Hz) indicated the configuration at this double bond to be *trans.* This assignment was confirmed by a NOESY experiment showing a positive NOE between H-4 and C*H*₃-1. The configuration at Δ^{5,6} was shown by the same experiment to be also *trans.* A positive NOE between H-4 and C*H*₃-6, and between H-5 and C*H*₂-8 was observed.

### Compound 3: (3E,5Z)-6-Methyl-octa-3,5-dien-2-one (example 4)

The large value of *J*_{H3-H4} (15.3 Hz) indicated the configuration at this double bond to be *trans.* This assignment was confirmed by a NOESY experiment showing a positive NOE between H-4 and C*H*₃-1. The configuration at Δ^{5,6} was shown by the same experiment to be *cis.* A positive NOE between H-5 and C*H*₃-6, and between H-4 and C*H*₂-7 was observed.

**Table 1: ¹H NMR data of compounds 1, 2 and 3 (δ in ppm, J in H_{Z})^{a}.**

| **Position** | **1** | **2** | **3** |
|---|---|---|---|
| 1 | 2.11 (3H, s) | 2.21 (3H, s) | 2.20 (3H, s) |
| 2 | - | - | - |
| 3 | 2.99 (2H, d, *J* = 7.3) | 6.01 (1 H, d, *J* = 15.3) | 6.00 (1 H, d, *J* = 15.3) |
| 4 | 5.10 (1 H, ttq, *J* = 7.3, 3.2, 2.6) | 7.37 (1H, dd, *J* = 15.3, 11.4) | 7.35 (1 H, dd, *J* = 15.3, 11.6) |
| 5 | - | 5.93 (1 H, brd, J = 11.4) | 5.89 (1 H, d, *J* = 11.6) |
| 6 | - | - | - |
| 7 | 1.88 (2H, dq, *J* = 7.4, 3.2) | 2.08 (2H, q, *J* = 7.5) | 2.26 (2H, q, *J* = 7.6) |
| 8 | 0.92 (3H, t, *J* = 7.4) | 1.00 (3H, t, *J* = 7.5) | 1.00 (3H, t, *J* = 7.6) |
| CH₃-6 | 1.63 (3H, d, *J* = 2.6) | 1.84 (3H, d, *J* = 0.8) | 1.82 (3H, s) |

| | | | |
|---|---|---|---|
| ^{a}s: singlet, d doublet, t triplet, q quartet, br broad | | | |

**Table 2: ¹³C NMR data of compounds 1, 2 and 3 (δ in ppm).**

| **Position** | **1** | **2** | **3** |
|---|---|---|---|
| 1 | 28.1 (CH₃) | 26.5 (CH₃) | 26.6 (CH₃) |
| 2 | 206.0 (C) | 197.8 (C) | 197.8 (C) |
| 3 | 43.7 (CH₂) | 127.4 (CH) | 127.2 (CH) |
| 4 | 82.8 (CH) | 138.7 (CH) | 138.0 (CH) |
| 5 | 201.6 (C) | 121.4 (CH) | 122.6 (CH) |
| 6 | 101.4 (C) | 151.9 (C) | 152.3 (C) |
| 7 | 25.9 (CH₂) | 32.1 (CH₂) | 24.9 (CH₂) |
| 8 | 11.2 (CH₃) | 11.2 (CH₃) | 12.1 (CH₃) |
| CH₃-6 | 17.8 (CH₃) | 16.4 (CH₃) | 23.0 (CH₃) |

### 2. Gaschromatography (GC area%)

### System

| | | |
|---|---|---|
| **Instrument:** | HP 6890 gas chromatograph with split injector and FID | |
| HP 6890 auto sampler | | |
| Data acquisition and reporting, HP ChemStation | | |
| **Inlet** | Split ratio | 75 : 1 |
| Injector temperature | 250°C | |
| Injection volume | 1µl | |
| Column | Stationary phase | Optima-1 |
| Length x diameter | 30m x 530µm; Film 3µm | |
| Column material | Fused silica | |
| Producer | Macherey Nagel | |
| Initial flow | 3.6 ml/min | |
| Mode | Constant flow | |
| **Column** | 50°C (10 min) → 6°C/min → 120°C (0 min) 10°C/min → 300°C (0 min). | |
| **temperature** | Total 40 min | |
| **Front Detector** | Detector temperature: | 300°C |
| Makeup Gas type | Helium | 45 ml/min |
| | Hydrogen flow | 40 ml/min |
| | Air flow | 450 ml/min |

## Claims

1. Process for the preparation of a compound of formula II 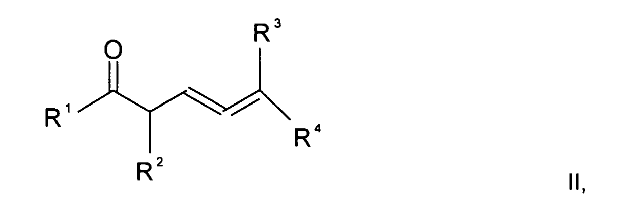 wherein R¹ is a C₁ - C₂₅ hydrocarbon moiety, and R², R³ and R⁴ are independently from each other H or a C₁ - C₂₅ hydrocarbon moiety,
which process comprises the step of reacting a compound of the formula III 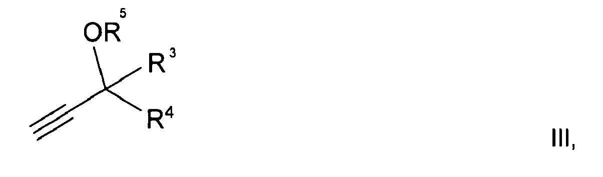 wherein R³ and R⁴ are defined as above and R⁵ is H,
with a compound of the formula IV 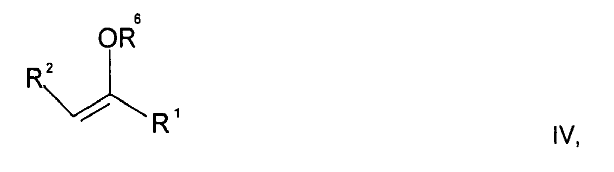 wherein R¹ and R² are defined as above and R⁶ is a hydroxy protecting group.

2. Process according to claim 1, wherein the reaction is conducted in the presence of an acid.

3. Process according to claim 2, wherein the acid is phosphoric acid or sulfuric acid.

4. Process according to any of the preceding claims, wherein R¹ is C₂ - C₆ alkyl and R² is H.

5. Process according to any of the preceding claims, wherein R³ is CH³ and R⁴ is C₂ - C₆ alkyl.

6. Process according to any of the preceding claims, which further comprises the step of isomerising a compound of the formula II 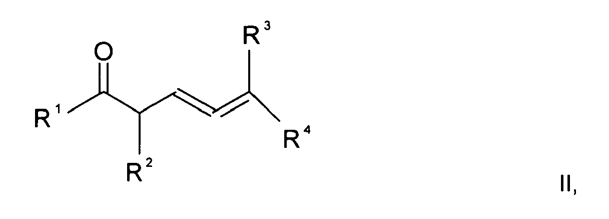 wherein R¹ is a C₁ - C₂₅ hydrocarbon moiety and R², R³ and R⁴ are independent from each other H or a C₁ - C₂₅ hydrocarbon moiety to obtain a compound of formula I 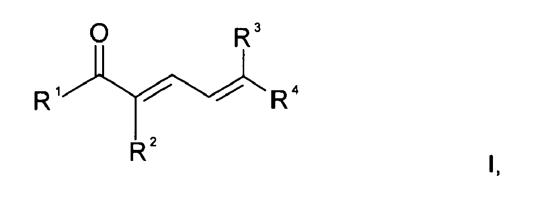 wherein R¹, R², R³ and R⁴ are defined as above.

7. Process according to claim 6, wherein the compound of formula II is purified before isomerisation.

8. Process according to claim 6, wherein the compound according to formula II is isomerised without prior purification.

9. Process according to any of claims 6-8, wherein the isomerisation is conducted under alkaline conditions.

10. Process according to any of claims 6-9, wherein R¹ is C₁ - C₆ alkyl and R² is H.

11. Process according to any of the claims 6 - 10, wherein R³ is CH₃ and R⁴ is C₂ - C₆ alkyl.

12. Process according to any of the preceding claims, wherein the compound of the formula II is 6-methyl-octa-4,5-dien-2-on.

13. Process according to any of the claims 6-12, wherein the compound of the formula I is 6-methyl-octa-3,5-dien-2-on.

14. Compound of formula V 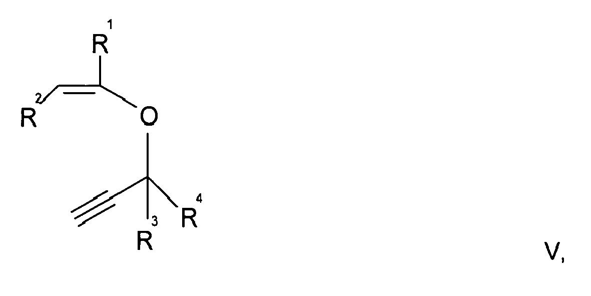 wherein R¹, R², R³ and R⁴ are defined as in claim 1.

15. Use of the compound of claim 14 for the preparation of dienones.

16. Use of the compound of claim 14, wherein R¹ is methyl, R² is hydrogen, R³ is methyl and R⁴ is ethyl for the preparation of 6-methyl-octa-3,5-dien-2-on.
